(19) Europäisches Patentamt
European Patent Office
Office européen des brevets

(11) Numéro de publication: **0 000 302**
**B1**

(12) # FASCICULE DE BREVET EUROPEEN

(21) Numéro de dépôt: **78400020.0**

(22) Date de dépôt: **14.06.78**

(51) Int. Cl.³: **C 07 D 453/04**

(54) **Nouveau procédé de préparation de la quinidine.**

(30) Priorité: **15.06.77 FR 7718397**
**15.06.77 FR 7718398**

(43) Date de publication de la demande:
**10.01.79 Bulletin 79/01**

(45) Mention de la délivrance du brevet:
**01.10.80 Bulletin 80/20**

(84) Etats contractants désignés:
**BE CH DE FR GB LU NL SE**

(56) Documents cités:
**DE - A - 1 165 604**
**FR - A - 2 100 618**
**FR - A - 2 332 278**
**NL - A - 7 612 951**

(73) Titulaire: **DEVINTER S.A.**
**2 Boulevard Royal**
**Luxembourg (LU)**

(72) Inventeur: **Bourrely, Jacques**
**4 avenue du Chateau**
**F - 92190 Meudon (FR)**

(74) Mandataire: **de Haas, Michel et al.**
**Cabinet Beau de Lomenie 55 rue d'Amsterdam**
**F - 75000 Paris 8ème (FR)**

**EP 0 000 302 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

Nouveau procédé de préparation de la quinidine

La présente invention se rapporte à un nouveau procédé de préparation de la quinidine, procédé impliquant notamment la réduction stéréospécifique de la quinidinone en quinidine.

La quinidine a été préparée depuis de nombreuses années par hémisynthèse à partir de la quinine. En effet, cet alcaloïde naturel du quinquina très utile dans le traitement des maladies cardiaques (comme anti-arrythmique n'est pas présent en quantité suffisante dans les écorces de quinquina pour assurer les besoins de l'industrie pharmaceutique.

Tous les procédés utilisés pour effecter l'hémisynthèse de la quinidine à partir de produits naturels sont des variations des réactions d'équilibres d'oxydo-réduction (oxydation d'OPPENHAUER — réduction de MEERWEIN-PONDORFF) (brevet allemand n° 883.154 (1953), n° 877.611 (1953).

En effet, en présence d'une cétone convenablement choisie (possédant un potentiel d'oxydation suffisant et une configuration moléculaire compatible avec les constantes d'encombrement stérique de la quinine et de quinidine), il s'établit un équilibre du type représenté sur la figure 1.

La figure 1 montre clairement qu'un produit de départ unique se trouve dans les conditions de l'équilibre, en présence de 4 produits principaux de réaction:

· la quinine et son isomère en $C_9$ épiquinine

· la quinidine et son isomère en $C_9$ épiquinidine

puis les intermédiaires de forme cétone en $C_9$

· quininone et quinidinone

qui, en solution, sont en équilibre passant par un état transitoire commun.

Le problème posé par cette hémisynthèse s'appréhende aisément: comment orienter l'équilibre vers la formation du produit désiré, à savoir la quinidine, tout en évitant surtout la formation de l'épiquinine et l'épiquinidine. Ces produits fatals sans utilisation thérapeutique ne peuvent pas, comme la quinine, être recyclés dans les conditions de la réaction. En effet, un mélange quinine + épiquinine ne donnera pas le même équilibre — toutes autres conditions étant maintenues égales — que la même quantité de quinine pure.

D'autre part, le présence d'épibases est indésirable dans les sels de quinidine pharmaceutiques et, de plus, la présence de ces isomères, même en faibles quantités, *provoque des retards* de cristallisation et des chutes de rendement importantes lors de la préparation des différents sels de quinine et de quinidine à partir de leurs solutions saturées.

En se maintenant au maximum à l'écart des conditions de formation des épibases, les principaux procédés de fabrication de quinidine sont ainsi parvenus à des coefficients de transformation qui ne sont qu'exceptionnellement inférieurs à 1,20 à 1,25 kg de quinine base anhydre engagée pour 1 kg de quinidine base anhydre isolée.

L'inconvénient majeur de ces synthèses est de nécessiter le recyclage d'environ 40 à 50% de la quinine engagée en des opérations ultérieures.

Même si la mise en réaction et l'obtention d'un équilibre satisfaisant peuvent être simplifiées, le fait de devoir recycler par opération la moitié de la charge de matière première entraîne bien évidemment des charges coûteuses dans les phases ultérieures de traitement des eaux mères, séparation de la quinine non transformée, purification avant remise en réaction.

Les frais financiers sont augmentés du fait du financement du ballast de quinine non transformé.

Le volume des réacteurs nécessaires et la charge de main-d'oeuvre sont plus importants que dans le cas d'une synthèse quantitative sans recyclages de matière première.

Récemment, plusieurs groupes de recherches ont utilisé certains hydrures d'aluminium dans la synthèse totale de séries d'alcaloïdes de la famille de la quinine et la quinidine.

En particulier, la réduction de la quinidone en quinidine a été décrite à l'aide de l'hydrure de diisobutylaluminium dans le toluène [Grethe, Uskokovic, JACS *93*, 5904 (1971)].

Cependent, l'utilisation seule de ce type de réactif n'est pas suffisante pour rentabiliser une synthèse industrielle; en effet, à côté de la quinidine, on retrouve les sous-produits déjà cités (quinine et épibases) en quantité inacceptable: 35% dans le cas de la référence citée, le rendement réel de quinidine isolée n'étant que de 65%.

De plus, la composition des sous-produits est en parts égales: la quinine, l'épiquinine et l'épiquinidine, soit 25% d'épibases non récupérables par opération, ce qui est totalement incompatible avec une économie rentable de production. Ayant repris ces travaux, on a tenté d'effectuer ces réductions à basse température, mais l'amélioration de rendement constatée n'équilibre pas le coût nécessaire à la production du froid.

La réduction d'une cétone par l'hydrure de diisobutylaluminium (DIBAH) s'effectue selon le schéma réactionnel suivant:

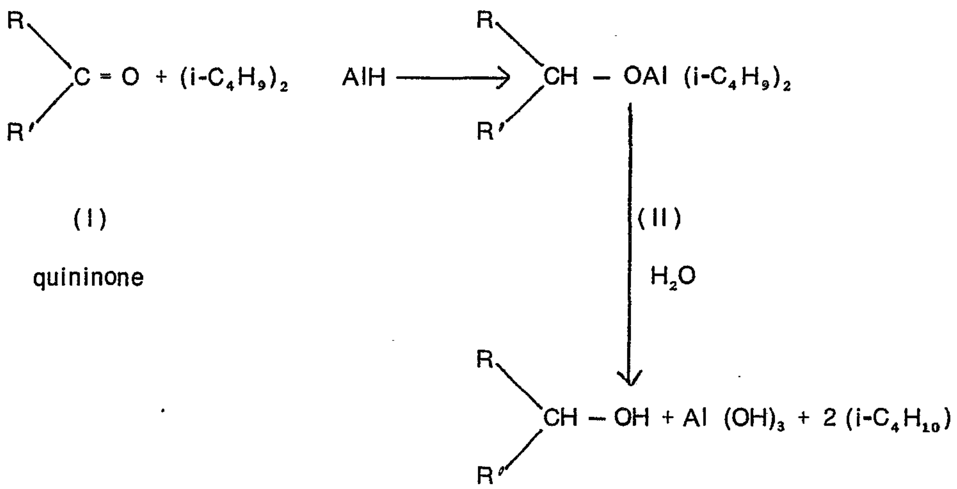


(I)

quininone

(III)

Dans le cas de la quinidine, du fait du carbone asymétrique en $C_9$, on peut imaginer que:

— soit deux complexes (II) stables sont susceptibles de se former (l'un correspondant à la forme quinidine, l'autre à la forme épibase),

— soit lors de l'hydrolyse du complexe un réarrangement peut avoir lieu orientant la formation de l'alcool secondaire (III) vers la forme quinidine ou épibase correspondante.

Il a été trouvé de façon très surprenante que, si la réduction de la quinidinone en quinidine, au moyen d'un hydrure d'aluminium ou d'un produit équivalent, était réalisée en présence d'une base organique hétérocyclique comportant un atome d'azote basique dans ledit hétérocycle, la réaction de réduction s'effectuait de façon entièrement stéréospécifique.

Le réducteur utilisé dans la présente invention est un hydrure d'aluminium, plus particulièrement un hydrure d'alkylaluminium ou un hydrure d'aluminium-sodium, plus particulièrement un hydrure d'alkylaluminium-sodium.

La base organique hétérocyclique utilisable dans la présente invention est de préférence la pyridine ou le pyrrole, les atomes de carbone de ces molécules hétérocycliques pouvant comporter un ou plusieurs substituants alkyles.

Les quantités de base organique cyclique à utiliser sont telles que la concentration de ladite base dans la solution où s'effectue la réaction soit comprise entre 0,5 et 100%.

La réduction a lieu dans un solvant tel qu'un solvant organique, le benzène, le toluène, le xylène ou un éther tel que le tétrahydrofuranne, le dioxanne ou le dibutyléther.

Mais la quinidinone ne constitue pas généralement le produit de base au départ duquel on effectue la semi-synthèse de la quinidine; ce produit de base est généralement la quinine. Il convient donc tout d'abord de transformer la quinine base en quinidinone avec le meilleur rendement possible. Il est possible selon l'invention d'utiliser n'importe quel procédé pour préparer la quinidinone à partir de la quinine base; il est cependant souhaitable que le procédé qui sera utilisé permette l'obtention d'une quinidinone pure.

Le procédé préféré pour obtenir la quinidinone pure utilisable comme réactif dans la réaction de réduction décrite précédemment consiste, à partir de la quinine base et à réaliser sur cette quinine, une réaction d'oxydation de type Oppenhauer au moyen d'un réactif basique résultant de la réaction d'une diphénylcétone sur un métal alcalin.

Comme diphénylcétone, on emploiera de préférence la benzophénone ou la fluorénone; comme métal alcalin, on utilisera par exemple du sodium ou du potassium. Le réactif basique est lui-même préparé par action du métal alcalin sur la cétone choisie en un milieu solvant qui est de préférence un milieu aromatique.

Dans de telles conditions, la quinine est oxydée de façon quasi-quantitative en quininone et quinidinone et la séparation par cristallisation de quinidinone du mélange obtenu permet de déplacer l'équilibre du milieu vers la préparation de la quinidinone.

Les exemples non limitatifs ci-après illustrent l'invention; les exemples 1 à 5 concernent le procédé de réduction de la quinidinone en quinidine et l'exemple 6 le procédé perfectionné préféré de préparation de la quinidinone à partir de la quinine.

Exemple 1

Dans un réacteur sec balayé à l'azote, on introduit:

— 500 ml de tétrahydrofuranne,

— 100 g de quinidinone cristallisée,
— 25 ml de pyridine anhydre.

En maintenant une température inférieure à 15°C, on coule 250 ml de solution de DIBAH à 25% dans le toluène.

En fin d'addition, un contrôle CCM indique que la réduction est terminée.

On effectue une distillation avec fractionnement (25 mm Hg) pour éliminer en tête le THF avec une quantité minimale de toluène.

Après refroidissement, on ajoute la quantité d'eau nécessaire pour décomposer le complexe aluminium avec un léger excès par rapport à la steochiométrie.

On filtre et on réextrait les cristaux avec du toluène à 80°C.

On obtient à partir de la solution toluénique 96 g de quinidine base, F. = 173°C, $\alpha_D$ (1,5% dans éthanol) = 256°C.

Exemple 2

On reproduit l'exemple 1 en utilisant les réactifs suivants:
— 600 ml de pyridine anhydre,
— 100 g de quinidinone cristallisée,
— 300 ml de solution de DIBAH à 25% dans le toluène.

La pyridine est chassée sous vide.

La masse réactionnelle est reprise dans 750 ml de mélange eau/éthanol 50/50, après reflux de 2 h et refroidissement, on obtient 92,7 g de quinidine base, F. = 174,5°C.

Exemple 3

On opère comme dans l'exemple 1 en utilisant les réactifs suivants:
— 300 ml de tétrahydrofuranne,
— 250 ml de pyridine anhydre,
— 100 g de quinidinone cristallisée,
— 250 ml d'une solution de DIBAH à 25% dans le toluène.

On a obtenu 94,5 g de quinidine ayant une température de fusion de 174°C.

Exemple 4.

On opère comme dans l'exemple 1 en utilisant les réactifs suivants:
— 500 ml de tétrahydrofuranne,
— 100 g de quinidinone cristallisée,
— 320 ml de $\gamma$ picoline anhydre,
— 250 ml d'une solution de DIBAH à 25% dans le toluène.

On a obtenu 89 g de quinidine.

Exemple 5

On a reproduit l'exemple 5, mais en utilisant, à la place de la $\gamma$ picoline, 200 ml de 3-méthyl-pyrrole.

On a obtenu 87 g de quinidine.

Exemple 6

On met en suspension 2,4 g de sodium dans 40 ml de xylène anhydre au reflux afin d'obtenir une bonne dispersion.

Après refroidissement à 90°C, on introduit lentement 36 g de benzophénone anhydre.

La solution prend alors la couleur bleu-vert, caractéristique du Ketyl (présence d'électrons).

Dans un second réacteur, on dissout à ébullition 13 g de quinine base anhydre dans 50 ml de xylène.

On coule cette solution dans la solution précédente. Après 60 min au reflux, la réaction est complète.

La solution xylénique est traitée à l'eau (20 ml) puis extraite par 100 ml d'acide sulfurique dilué à 20%.

On neutralise par addition d'ammoniaque la solution sulfurique froide.

Il se sépare une huile qui cristallise lentement après ensemencement.

Les cristaux de quinidinone sont séparés du mélange.

Poids obtenu: 12,2 g (94% de la théorie)

Chromatographie en couches minces

Eluant acétone 80/DMF 20 1 Tache Rf = 0,8

**Revendications**

1. Procédé de préparation de quinidine à partir de quinine par oxydation et réduction de la quinidinone formée, caractérisé en ce que la réduction est effectuée grâce à l'action d'un hydrure d'aluminium en présence d'une base organique hétérocyclique comportant un atome d'azote basique dans ledit hétérocycle.

2. Procédé selon la revendication 1, caractérisé en ce que l'hydrure d'aluminium est choisi parmi les hydrures d'alkylaluminium, plus particulièrement les hydrures de dialkylaluminium et les hydrures d'alkylaluminium-sodium.

3. Procédé selon l'une des revendications 1 et 2, caractérisé en ce que ladite base hétérocyclique est choisie parmi la pyridine, le pyrrole et les divers alkyl-substitués, sur les carbones, de ces molécules.

4. Procédé selon l'une des revendications 2 et 3, caractérisé en ce que l'hydrure d'aluminium est le diisobutylaluminium hydrure.

5. Procédé selon l'une des revendications 1 à 4, caractérisé en ce que ladite base est la pyridine et qu'elle est utilisée à une concentration supérieure à 0,5%.

6. Procédé selon l'une des revendications 1 à 4, caractérisé en ce que l'hydrure est utilisé sous forme d'une solution dans un solvant aromatique ou dans un éther, tel que le tétrahydrofuranne, le dioxanne et le dibutyléther.

7. Procédé selon l'une des revendications 1 à 6, caractérisé en ce que la quinidinone est utilisée sous forme d'une solution de cette molécule dans un solvant choisi parmi la pyridine et en mélanges de pyridine avec un éther, tel que le tétrahydrofuranne, le dioxanne et les dialkyléthers.

8. Procédé selon la revendication 1, caractérisé en ce que la quinidinone de départ est obtenue par une réaction d'oxydation, de la quinine, de type Oppenhauer au moyen d'un réactif résultant de la réaction d'une diphénylcétone, comme par exemple la benzophénone et la fluorénone sur un métal alcalin.

**Claims**

1. A process for the preparation of quinidine from quinine by oxidation and reduction of the resulting quinidinone, characterized in that the reduction is effected owing to the action of a aluminium hydride in the presence of an organic heterocyclic base containing a basic nitrogen atom in said heterocycle.

2. A process according to claim 1, characterized in that the aluminium hydride is selected from the alkylaluminium hydrides, more particularly from the dialkylaluminium hydrides and the alkylaluminium-sodium hydrides.

3. A process according to anyone of claims 1 and 2, characterized in that said heterocyclic base is selected from pyridine, pyrrole and the different alkyl-substitutes, on the carbons, of these molecules.

4. A process according to anyone of claims 2 and 3, characterized in that the aluminium hydride is the diisobutylaluminium hydride.

5. A process according to anyone of claims 1 to 4, characterized in that said base is pyridine and that it is used at a concentration greater than 0,5%.

6. A process according to anyone of claims 1 to 4, characterized in that the hydride is used in the form of a solution in an aromatic solvent or in an ether, such as tetrahydrofurane, dioxane and dibutylether.

7. A process according to anyone of claims 1 to 6, characterized in that the quinidone is used in the form of a solution of this molecule in a solvent selected from pyridine and mixtures of pyridine with an ether, such as tetrahydrofurane, dioxane and the dialkylethers.

8. A process according to claim 1, characterized in that the starting quinidinone is obtained by an Oppenhauer type oxidation reaction of quinine, by means of a reagent resulting from the reaction of a diphenylketone, such as for example benzophenone and fluorenone, with an alcaline metal.

**Patentansprüche**

1. Verfahren zur Herstellung von Chinidin aus Chinin durch Oxydierung und Reduktion des gebildeten Chinidinons, dadurch gekennzeichnet, dass die Reduktion dank der Wirkung eines Aluminium-Hydrids in Gegenwart einer organischen heterozyklischen Base durchgeführt wird, welche in dem genannten Heterozyklus ein basisches Stickstoffatom enthält.

2. Verfahren nach Anspruch 1, dadurch gekennzeichnet, dass das Aluminium-Hydrid unter den Alkylaluminium-Hydriden, insbesondere den Dialkylaluminium-Hydriden und den Natrium-Alkylaluminium-Hydriden gewählt wird.

3. Verfahren nach einem der Ansprüche 1 und 2, dadurch gekennzeichnet, dass die genannte heterozyklische Base unter Pyridin, Pyrrol und ihren verschiedenen Derivaten gewählt wird, die auf Kohlenstoffatomen alkylsubstituiert sind.

4. Verfahren nach einem der Ansprüche 2 und 3, dadurch gekennzeichnet, dass das Aluminium-Hydrid das Diisobutylaluminium-Hydrid ist.

5. Verfahren nach einem der Ansprüche 1 bis 4, dadurch gekennzeichnet, dass die genannte Base das Pyridin ist und dass sie als Konzentration von mehr als 0,5% benutzt wird.

6. Verfahren nach einem der Ansprüche 1 bis 4, dadurch gekennzeichnet, dass das Hydrid in Form einer Lösung in einem aromatischen Lösungsmittel oder in einem Äther, zum Beispiel Tetrahydrofuran, Dioxan und Dibutyläther, benutzt wird.

7. Verfahren nach einem der Ansprüche 1 bis 6, dadurch gekennzeichnet, dass das Chinidinon in Form einer Lösung dieses Moleküls in einem Lösungsmittel benutzt wird, das unter Pyridin und den Mischungen von Pyridin mit einem Äther, wie Tetrahydrofuran, Dioxan und den Dialkyläthern gewählt wird.

8. Verfahren nach Anspruch 1, dadurch gekennzeichnet, dass das Ausgangs-Chinidinon durch eine Oxydierungsreaktion des Chinins vom Typ Oppenhauer erhalten wird, mittels eines aus der Reaktion eines Diphenylketons, wie zum Beispiel Benzophenon und Fluorenon, mit einem Alkylmetallentstehenden Reagens.

FIGURE 1

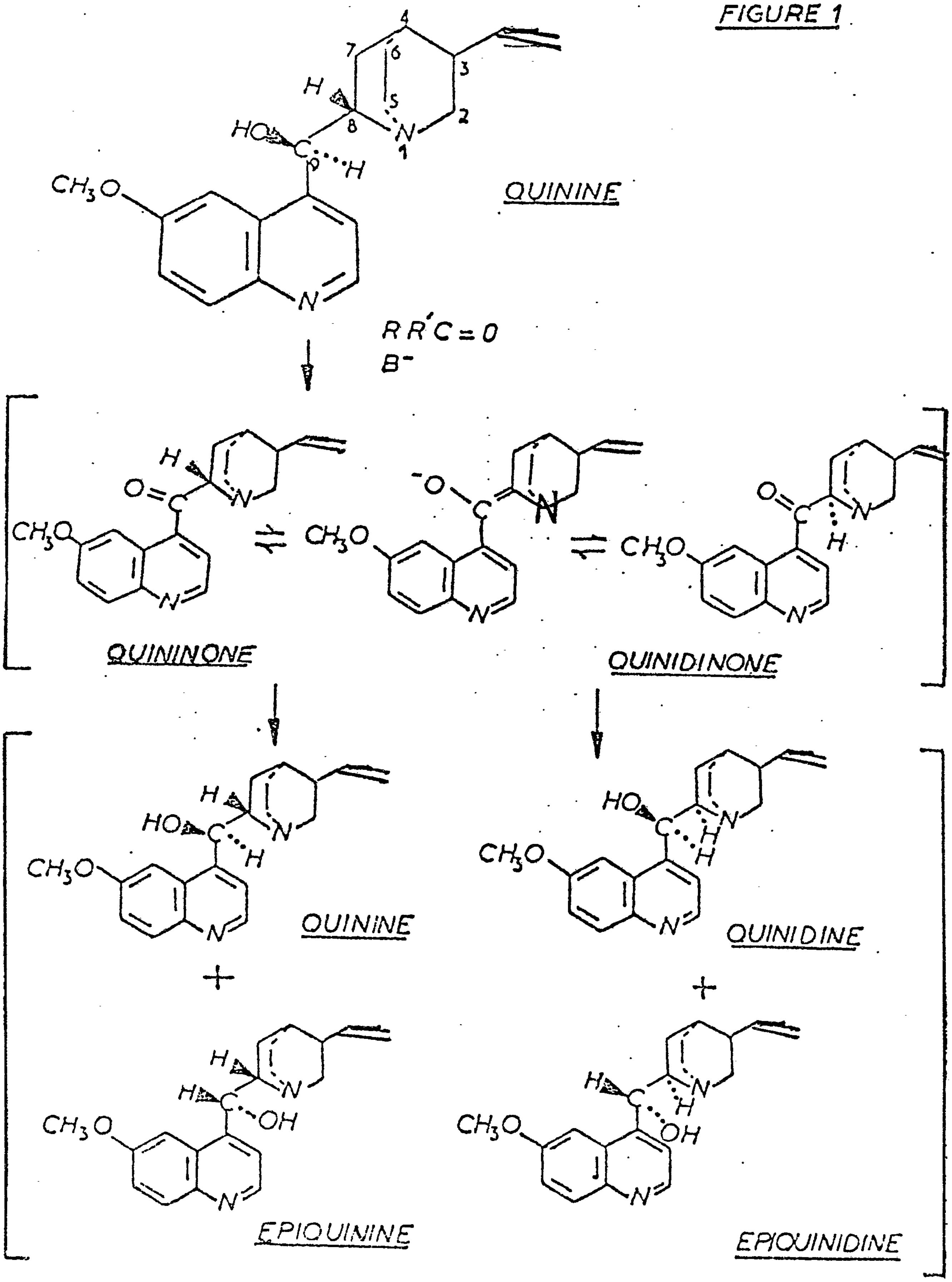


QUININE

QUININONE

QUINIDINONE

QUININE

QUINIDINE

+

+

EPIQUININE

EPIQUINIDINE